# EUROPEAN PATENT APPLICATION

(11) **EP 1 082 946 A1**
(43) Date of publication of application: **14.03.2001**
(21) Application number: 99890289.4
(22) Date of filing: 09.09.1999
(51) Int. Cl.: A61F 2/06

(54) **Intraluminal graft with variable internal diameter**

(71) Applicant: Grampp, Stephan, Dr. med., 1180 Wien (AT); Henk, Christine, Dr. med., 1180 Wien (AT)
(72) Inventor: Grampp, Stephan, Dr.med., 1180 Wien (AT); Henk, Christine, Dr.med., 1180 Wien (AT); Lammer, Johannes, Prof.Dr.med., 1230 Wien (AT)

(57) **Abstract**

An intraluminal graft (stent), with variable inner diameter is placed into the body by means of a catheter or introduction set. An inflatable, balloon-like structure fixed to the inner surface of the graft can be expanded by access through a port on the outer surface of said graft. By applying a volume of an agent of any sorts into said port, the inflatable balloon attached to the inner diameter of the graft can be expanded to meet and/or improve various physiologic and pathologic conditions of the host. The expansion can be of further use to support cleaning of the inner aspect of the graft by loosening clotted residual fluids or other adhesive materials.

## Description

### BACKGROUND - FIELD OF THE INVENTION

Grafts for angioplasty of blood vessels or for use in other passageways such as bile ducts or the gastrointestinal tract have largely proved useful in medical practice for the prevention of occlusions or re-stenosis after therapy. Known grafts do for example consist of cartridge-shaped lattice of steel or plastic, which are self-expanding or balloon-expandable. The invention relates to an intraluminal graft for use within an intracorporal passageway or duct and a method for adjusting and changing the internal diameter of the graft by manipulation from outside.

### BACKGROUND - DESCRIPTION OF PRIOR ART

This discovery concerns a graft, stent, or endoprosthesis, which will further be referred to solely under the term 'graft' and an apparatus for percutaneous manipulation of the graft as per the general description given in the description of the invention.

There are different kinds of stents or endoprostheses commonly called grafts, which have the common characteristic of being presented into a blood vessel, or other body cavity, or lumen via endoscopic or fluoroscopic controlled manipulation, usually by means of a guide wire. Grafts can for example consist of a cartridge-shaped lattice made of steel or of a wire mesh, or of other materials and may be porous or may be covered with an isolating, impenetrable material.

Grafts of various kinds and sizes have largely proved useful in medical practice for the therapy or prevention of occlusions or re-stenosis after transluminal placement in the blood vessels, the biliary tract, the airways, the gastrointestinal tract, or the urogenital system. Occlusion of body passages or ducts may occur by clotting of the fluids or substances transported to or through those, by tissue growth due to malignant or benign conditions, by development of scar tissue, or by compression from outside.

Intraluminal grafting has been demonstrated to present an alternative to conventional surgical approaches. It was most successfully applied in intravascular grafting, which involves the percutaneous insertion of a graft into a blood vessel and its delivery by means of a catheter to its desired location. In many cases the graft may be expanded to widen the lumen of a body passageway as for example described in U.S, Pat. 4,733,665. Other body passageways may be accessed through natural openings on the body surface or through artificial percutaneous access. In most of the approaches, grafts were expanded through use of a catheter mounted balloon. In these procedures the balloon is inflated within the stenosed structure or within a newly drilled or cut passageway through tissue.

Structures which have been previously used as intraluminal grafts have included coiled steel springs, helically wound coil springs, or expanding steel grafts in a zig-zag pattern. The inner as well as outer diameters of those mostly cylindrical structures were defined either by manufactured design specifications or by applying pressure to their inner surface upon installation and expanding the graft to a desired size.

In general, the foregoing structures have one major disadvantage in common. These structures must be delivered to the desired location in a collapsed state and then expanded to their final size and shape by either a spring mechanism, mechanical pressure via a balloon catheter or through built-in elastic memory by changing their temperature. When finally placed, those implants will not change their shape and especially can not change the diameter of their inner passage any more. The change of the inner diameter of a graft however may be a therapeutical requirement for certain medical conditions such as intrahepatic portosystemic shunts, where a graft allows the blood from the portal vein circumvent the liver and reach the inferior vena cava. In those cases, a preoperative planning of the hemodynamic properties and requirements is usually not possible. This therapeutic intervention can result in a throughput of blood through the shunt which is too high and needs to be decreased. Thus, conventional grafts do not necessarily provide an acceptable long-term solution in the face of progredient disease. In those cases, only a total change of the graft followed by a re-implantation of a new graft with smaller inner diameter or overgrafting with a smaller graft could be performed until now.

Additional disadvantages associated with conventional grafting are the high likelihood that those grafts do in a considerable percentage of cases clot from fluids passing through the graft. This may be for example the clotting of blood on the inner surface of the material or accordingly the adhesion of bile and other materials in the various applications of intracorporal grafting.

Prior to the development of the present invention, there has been no intraluminal graft, and method, and apparatus for changing or adjusting the lumen of a body passageway. Therefore, the art has sought an intraluminal graft, and method, and apparatus for changing the lumen of a body passageway, which is believed to be able to be used in such conditions as intrahepatic portosystemic shunts, or for biological and medical experiments requiring the partial or total obstruction of a vessel or any other passageway.

### SUMMARY OF THE INVENTION

This invention relates to devices known as grafts which provide support to a body passageway, which can be a natural passageway such as a blood vessel, or can also be an artificial passageway following a therapeutic intervention. It relates further to a method and a system for varying the inner diameters of an intraluminal graft.

The present invention is summarized in that a specific designed graft is placed into a body by means of a catheter, introduction set, or surgical manipulation. The graft is for example a hollow, cylindrical (tube-like) structure made of a synthetic substance or can be also a wire mesh tube covered with a synthetic substance, or a wire mesh. The graft may for example be self-expanding as for example described in U.S, Pat. 5,061,275 or be balloon-expandable as for example described in U.S, Pat. 4,733,665. The structure provides stability for maintaining the size of a body passageway, thereby preventing constriction and maintaining the lumen open. The device may for example be transported and introduced into the target area by means of a catheter, a guide wire, or an endoscope. A balloon-like, ring-shaped or cushion-shaped structure fixed to the inner diameter of the graft can be expanded by access through a port on the outer surface of said graft. The present invention includes a tubular-shaped graft with an inner inflatable, expandable portion. A port is attached to the outer surface of the tubular-shaped graft to enable manipulation of the inner portion from outside. Expansion of the inner, inflatable structure can be performed by percutaneous manipulation with specially designed tools. For temporary use, such a tool may consist of a percutaneous punction needle or specifically designed access tool which can be linked to a port at the outer surface of said graft. For long-term use, such a tool may consist of a subcutaneous port, which may be permanently linked to a port at the outer surface of said graft. By applying a volume of gas, liquid, or any other suitable agent into said port, the balloon attached to the inner diameter of the graft can be expanded. By choosing the appropriate volume of expansion, the inner diameter of the graft can be adjusted meeting various physiologic and pathologic conditions of the host. The motion which goes along with the expansion or the reverse motion of deflation can be of further use to support cleansing of the inner aspect of the graft by loosening clotted residual fluids, which may be adhesive to the graft.

A method of the present invention comprises the steps of: inserting an intraluminal graft, which may be for example disposed upon a catheter, into a body passageway into a desired location. A feature of the present invention is that the variable inner diameter may be adjusted by manipulation from outside the body passageway where it is hosted. This results in a certain permanent pressure within the inflatable cushion, which has to be sufficiently large to restrict the flow of the fluids passing through the passageway. It is an object of the present invention to provide means of gradually or totally obstructing a lumen. This is may be particularly useful for a variety of medial or biological experiments.

Another feature of the present invention is that if the inflatable cushion is expanded, it may possibly break, crack, and remove particles of thickened or clotted materials adjacent to the surface of the graft. Those adhesive materials may be for example clotted blood or bile, may partially or totally obstruct the lumen.

It is another object of the present invention to provide a intraluminal graft of the character above which can be readily and economically manufactured.

Other objects, advantages, and features of the present invention will become apparent from the following specification when taken in conjunction with the accompanying drawings. When the invention will be described in connection with the preferred embodiment, it will be understood that it is not intended to limit the invention to that embodiment. On the contrary, it is intended to cover all alternatives, modifications, and equivalents as may be included within the spirit and scope of the invention as defined by the appended claims.

### BRIEF DESCRIPTION OF THE DRAWINGS

FIG. 1. A perspective view of a graft for a body passageway with an expandable inner balloon.

FIG. 2. An axial cross-sectional view of a graft for a body passageway with an expandable inner balloon.

FIG. 3. A longitudinal cross-sectional view of a graft with an expandable inner balloon before inflation.

FIG. 4. A longitudinal cross-sectional view of the port of a graft with an expandable inner balloon.

FIG. 5 a. A longitudinal cross-sectional view of a graft with an expandable inner balloon after inflation. Inner surface of the expandable inner balloon parallel to the cuff.

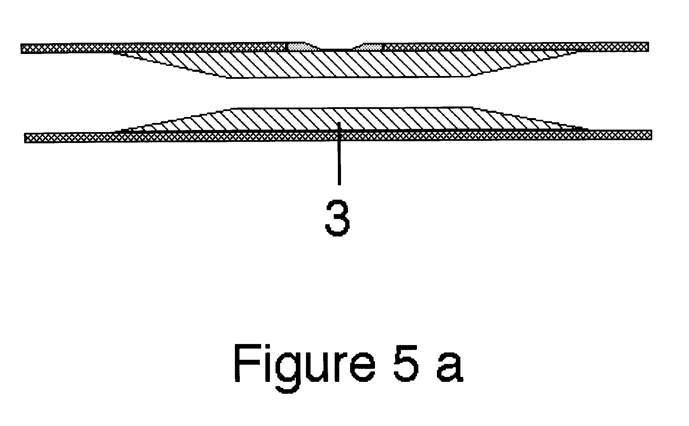

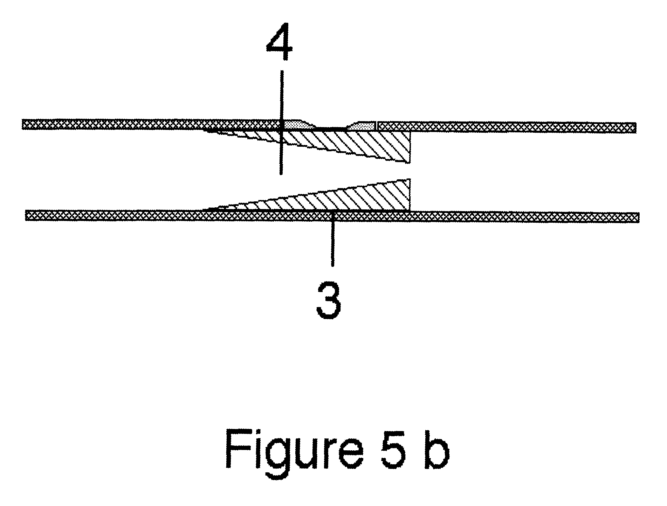

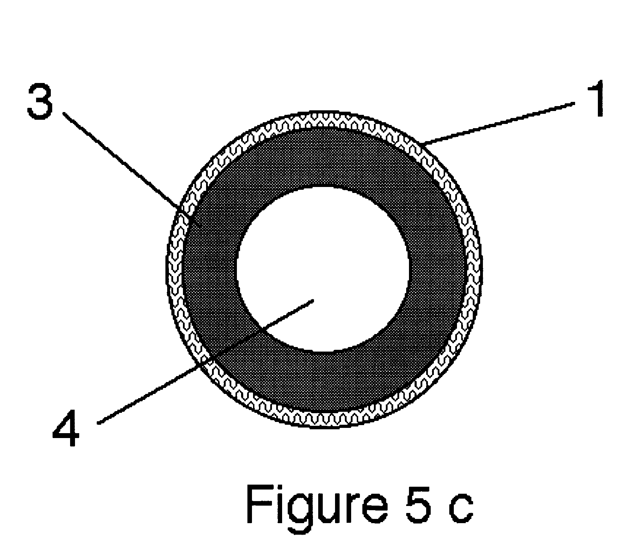

FIG. 5 b. A longitudinal cross-sectional view of a graft with an expandable inner balloon after inflation. Inner surface of the expandable inner balloon wedge-shaped lays in a distinct angle to the longitudinal axis of the cuff.

FIG. 5 c. An axial cross-sectional view of a graft with an expandable inner balloon after inflation.

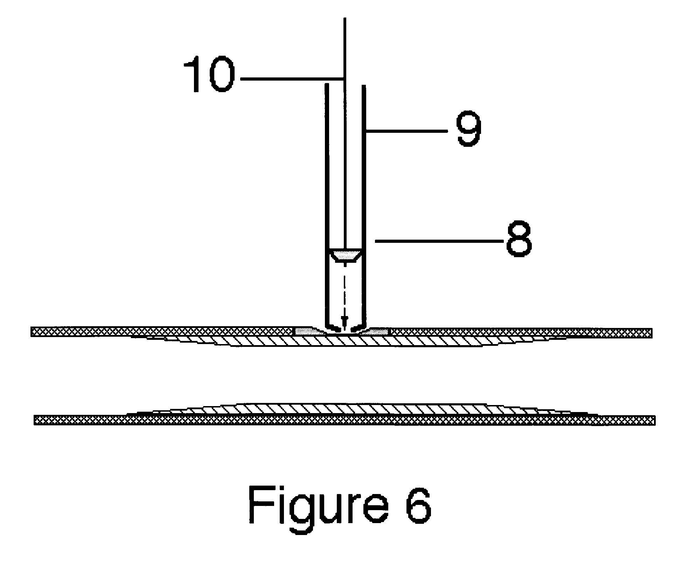

FIG. 6. A longitudinal cross-sectional view of a graft with an expandable inner balloon before inflation pictured with an access tool (apparatus for inflation) attached.

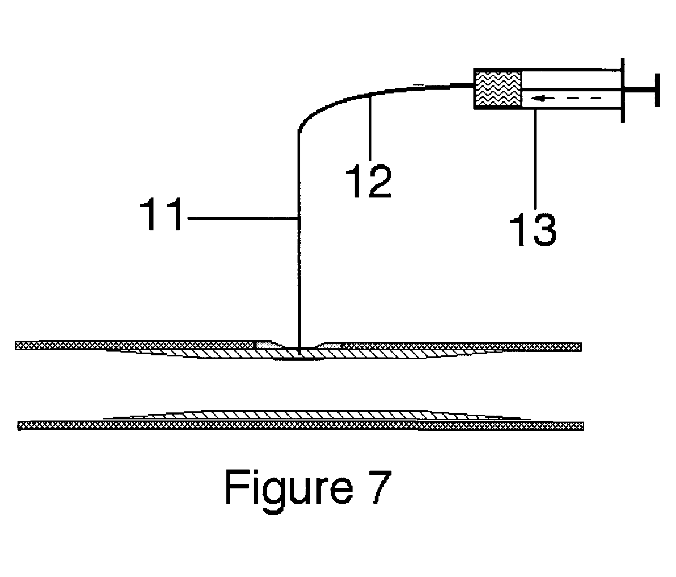

FIG. 7. A longitudinal cross-sectional view of a graft with an expandable inner balloon before inflation pictured with an injection needle attached.

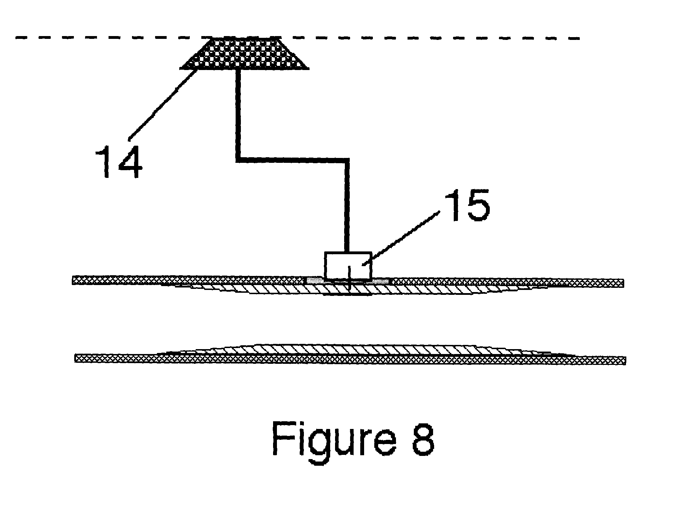

FIG. 8. A longitudinal cross-sectional view of a graft with an expandable inner balloon in deflated condition with a subcutaneous port system attached.

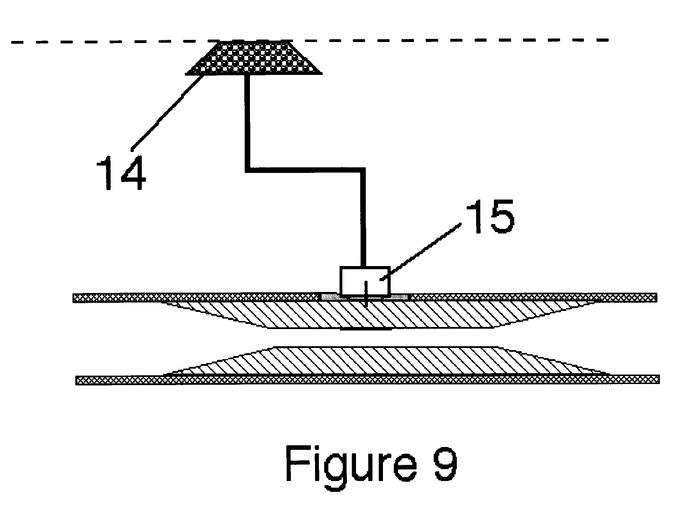

FIG. 9. A longitudinal cross-sectional view of a graft with an expandable inner balloon after inflation with a subcutaneous port system attached.

### DETAILED DESCRIPTION OF THE INVENTION

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further applications of the principles of the invention as illustrated therein being contemplated as would normally occur to one skilled in the art to which the invention relates.

The invention is directed in particular towards manipulation of the internal diameter of body passageways maintained by a graft. It is further directed towards a change in the morphology of the inner graft surface to facilitate or cause the detachment of clotted substances, which may contribute to the obstruction of the inner lumen.

The use of the words prosthesis, endoprosthesis, graft, and stent encompasses the usages within various types of body passageways.

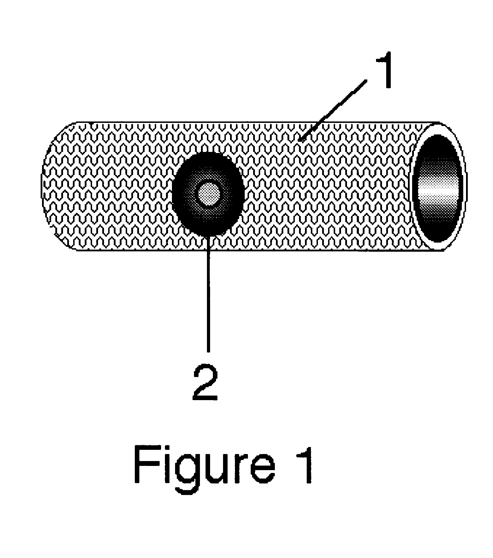

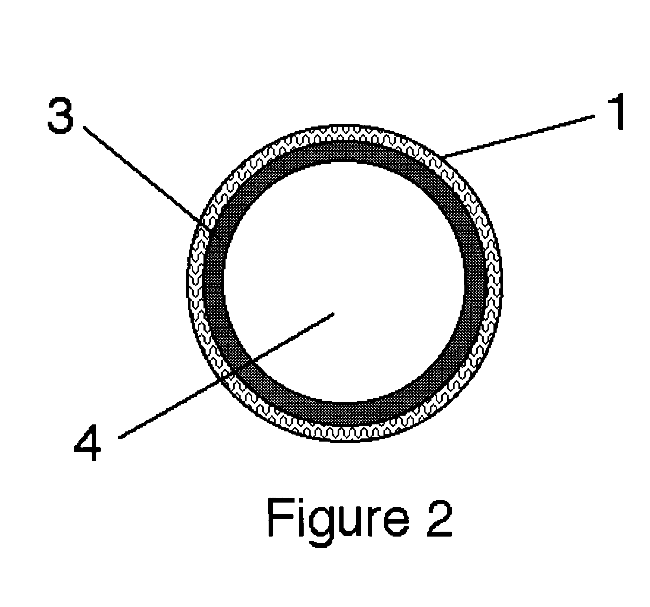

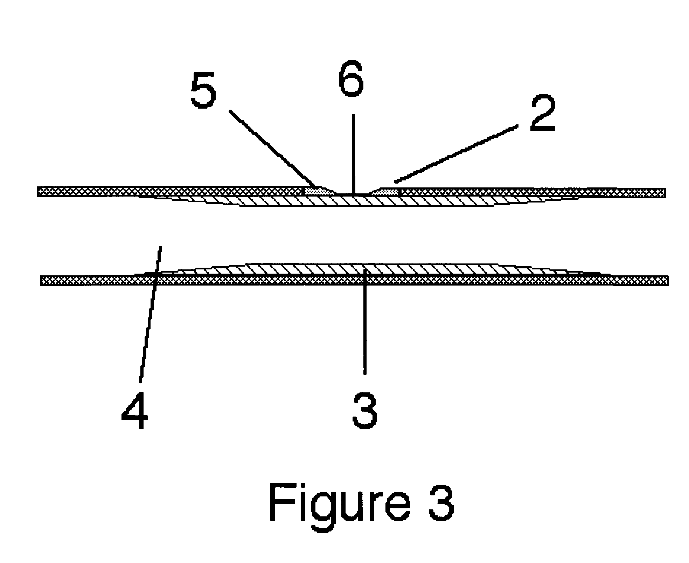

Shown in figures 1 and 2 is a graft, which implements the present invention consisting of a cuff or outer hull 1 made for example of a wire mesh, which may or may not be covered by a surface coating of material, which may have anti-thrombogenic properties (such as carbon fiber). Many of the details of how such outer cuffs are designed and produced are described in patent applications. Specifications are hereby incorporated by reference. The cuff 1 has one or more ports 2 penetrating its surface as shown in figure 3. This port 2 penetrates the cuff 1 and is used as the link between tools for outer manipulation and the inflatable cushion or inner balloon 3, which is attached to the inside of the cuff 1. The inner balloon 3 accordingly represents the outer boundary of the lumen 4 maintained by the graft. The inner balloon 3 is attached to the outer cuff by stitching, gluing, or any other method sufficient to ensure bonding. If the cuff 1 were made of plastic, the balloon could also be attached to the cuff by embedding in the plastic; it being understood that the means for attaching the cuffs to the balloon can be varied depending on the cuff material and other factors without diverging from the intended scoop of the present invention. The inner balloon 3 may have an intraluminal surface which, when inflated is directed parallel to the cuff 1 or may also be wedge-shaped in the cross sectional longitudinal view resulting in an intraluminal surface which, when inflated is directed in a distinct angle to the longitudinal axis of the cuff 1.

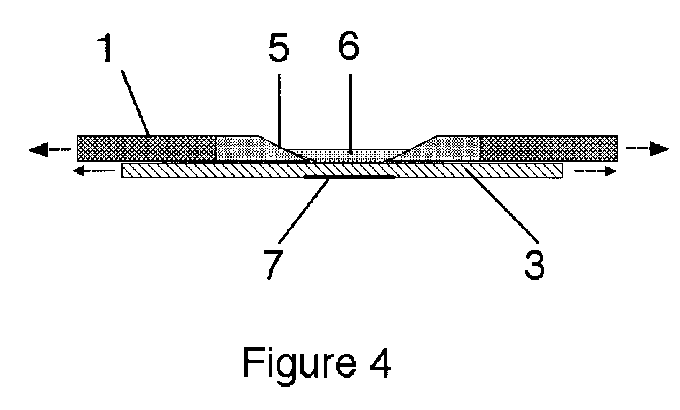

As demonstrated in figure 4, the port 2 may consist of a funnel-shaped ring 5, for example made of metal or plastic, which is embedded into or attached onto the cuff 1. The opening inside the ring 5 is covered by a self-sealing member 6, which is attached to the inner aspect of the ring 5. Such a member is for example described in U.S, Pat. 4,840,615. Access to the inner balloon 3 is possible through the port 2 by penetrating the surface member 5 of the port 2 with a injection needle or specific access tool. In cause of use of a needle or other tool with a sharp or pointed surface the inner aspect of the inner balloon 3 may potentially be in danger of being penetrated. Therefore the inner aspect of the inner balloon 3 may be strengthened and/or protected by a metal or plastic plate 7 attached directly below the port 2. A needle or other pointed object may now dislocate the inner aspect of the inner balloon 3 into the lumen but will not penetrate the fabric of the inner balloon 3. Expansion of the inner balloon 3 of the graft inside the host body as demonstrated in figure 5 a, 5 b, and 5 c may be accomplished from outside by means of manipulation through a punction tool, injection-type hollow needle, or permanent connection. In this embodiment a possible punction or access tool 8 as shown in figure 6 comprises of a tube-like catheter 9 for example made from metal, which enables the percutaneous punction through tissue. The access tool 8 may for example be used under fluoroscopic control and connects to the port 2 on the outer surface of the cuff 1. Said port 2 has for example an outer rim or ring 5 to guide the punction tool to its desired location, which usually will be in the center of the port 2. The access tool also comprises further of an inner portion which is a guided hollow needle-like structure 10 and fits inside of the outer hull of the punction tool. The hollow needle-like structure 10 is constructed in a fashion that if it is placed inside the outer hull, its tip reaches an appropriate amount outside of the end of said outer hull to penetrate the member 6. The distance between the end of the tube 9 and the end of the inserted needle 10 may be suited to penetrate through the outer connecting member 6 of the port 2 and reach the internal cavity of the inner balloon 3. Through the needle 10 of the access tool 8 or through a regular injection needle 11, connected for example by means of a tube 12 to a syringe 13 or any other device suited for creating pressure, an agent may be injected into the inner balloon 3. This will lead to an expansion and inflation of the inner balloon 3 as shown in figure 5. Access by an injection-type needle is depicted in figure 7.

Expansion of the inner balloon 3 of the graft 1 inside the host body as demonstrated in figure 5 may, as mentioned, also be accomplished by means of a permanent connection as shown in figure 8. In this embodiment, a possible subcutaneous port 14 comprises of a cushion-like structure with a surface member, which may be accessed through percutaneous puncture with an injection-needle like device. Such a subcutaneous port 14 is for example described in U.S, Pat. 4,840,615. The subcutaneous port 14 may be connected to the port 2 on the outer surface of the cuff 1 with a tube-like catheter 12. The tube-like catheter 12 may be connected to the port 2 on the outer surface of the cuff 1 by means of a connecting part 15, which docks into the cavity of the port 2 and may have an attached hollow needle on its surface to penetrate the member 6. Through a regular injection needle 11, which might be connected to a syringe 13 or any other device suited for creating pressure a fluid or a gas may be injected into the subcutaneous port 14, which is connected to the inner balloon 3 in order to expand the inner balloon 3 as shown in figure 9. Installation of such a subcutaneous port 14 may for example be performed under fluoroscopic control or by open surgical access.

In order to provide means by which the position of the graft or cuff 1, especially the position of the port 2 or ports may be determined by fluoroscopic devices, one or more radioopaque markers may be located on the graft and in the port or ports; this may be for example a radioopaque band around the connecting member 6.

In order to provide means by which the position of the graft or cuff 1, especially the position of the inner balloon 3 and its amount of inflation e.g. size of the inner diameter can be determined, one or more radioopaque markers may be located on the inner surface of said inner balloon 3; this may be for example in a ring shaped structure which can be visualized in an axial direction by means of fluoroscopic diagnosis. It is to understood that the present invention is not to be interpreted to be limited to use under fluoroscopic control but also to use controlled by other diagnostic modalities such as ultrasound, computed tomography, or magnetic resonance imaging. The material properties of the graft 1 as well as of potential markers or injected fluids or gases which support in determining the desired location of graft 1 and/or port 2 or of the status of inflation of the inner balloon 3 may be chosen according to different imaging requirements; these may for example be materials, which are susceptible to magnetic fields as used in magnetic resonance imaging, x-rays, or ultrasonic waves.

Using our said approach the instrument is primarily capable of providing variable inner diameters in a graft and to provide also dynamic movement of the inner surface of said graft.

It is to understood that the present invention is not limited to the exact details of construction, operation, exact materials or embodiment shown and described, as obvious modifications and equivalents will be apparent to one skilled in the art. Instead it encompasses all variations thereon which come within the scope of the following claims.

### REFERENCES CITED

| U.S. PATENT DOCUMENTS | | |
|---|---|---|
| 4,733,665 | 3/1988 Palmaz | 128/343 |
| 4,832,690 | 5/1989 Kuu | 604/85 |
| 4,840,615 | 6/1989 Hancock | 604/93 |
| 5,061,275 | 10/1991Walsten | 623/1 |
| 5,147,385 | 9/1992 Beck | 623/1 |

| EUROPEAN PATENT DOCUMENTS | |
|---|---|
| 93250022.6 2/1987 | European Pat. Off. A 61F 2/06 |
| 88309274.4 10/1988 | European Pat. Off. A 61F 2/06 |
| 90101509.9 1/1990 | European Pat. Off. A 61F 2/06 |
| 91309197.1 10/1991 | European Pat. Off. A 61F 2/06 |
| 92305455.5 6/1992 | European Pat. Off. A 61F 2/06 |

### OTHER PUBLICATIONS

Becker,C.D. et al. "Percutaneous palliation of malignant obstructive jaundice with the Wallstent endoprosthesis." *JVIR* 4: 597 (1993)
Culp,W.C. et al. "Biliary strictures in liver transplant recipients: treatment with metal stents." *Radiology* 199: 339 (1996)
Jackson,J.E. et al. "Biliary endoprosthesis dysfunction in patients with malignant hilar tumors: successful treatment by percutaneous replacement of the stent." *Am J Roentgenol* 155(2): 391 (1990)
Hausegger,K.A. et al. "Benign biliary obstruction : is treatment with the Wallstent advisable ?" *Radiology* 200: 437 (1996)
Kishi,K. et al. "Dacron-covered stent therapy for portal vein tumor thrombus in hepatocellular carcinoma." *Acta Radiol* 34: 266 (1993)
Kubota,Y. et al. "Bilateral internal biliary drainage of hilar cholangiocarcinoma with modified Gianturco Z stents inserted via a single percutaneous tract." *JVIR,* 4: 605 (1993)
Palmaz,J.C. et al. "Intravascular stents: tissue-stent interactions and design considerations." *Am J Roentgenol ,* 160: 613 (1993)
Peltzer,M.Y. et al. "Treatment of Budd-Chiari syndrome with a transjugular intrahepatic portosystemic shunt. *" JVIR ,* 4: 263 (1993)
Rossi,P. et al. "Metallic stents in malignant biliary obstruction: results of a multicenter European study of 240 patients." JVIR, 5: 279 (1994)
Rousseau,H. et al. "Transjugular intrahepatic portosystemic shunts using the Wallstent prosthesis: follow-up study." *Cardiovasc Intervent radiol,* 17: 7 (1994) Sapoval,M.R. et al. "Outcome of percutaneous intervention in iliac artery stents" *Radiology* 198: 481 (1996)
Vorwerk,D. et al. "Primary stent placement for chronic iliac artery occlusions: follow-up results in 103 patients." *Radiology* 194: 745 (1995)
Vorwerk,D. et al. "Venous stenosis and occlusion in hemodialysis shunts: follow-up results of stent placement in 65 patients." *Radiology* 195: 140 (1995)

## Claims

1. A graft comprising a tubular body with an inner and outer surface being open on both ends and defining a diameter with a balloon-like, expandable structure fixed to its inner wall, whose outer wall may be of a solid material, or a mesh made of any material.

2. A graft according to claim 1, wherein the expandable or deflatable inner structure is wedge shaped, double wedge shaped, or oval in the cross-sectional aspect, or has any other shape suited for its purpose.

3. A graft according to claims 1 to 3, wherein the expandable or deflatable inner structure is at one or several places aligned to the inner wall of the graft, and/or may be circular around the inner circumference.

4. A graft according to claims 1 to 3, wherein the expandable or deflatable inner structure has any other conceivable shape in the cross-section suited for downsizing the inner orifice in various degrees or totally blocking it.

5. A graft according to claims 1 to 4, wherein the expandable or deflatable inner structure can be inflated or deflated by manipulation from outside of the passageway or the body where said graft is placed.

6. A graft according to claims 1-5, wherein the inner cushion is accessible through a port, which itself is accessible by means of a access device, injection needle, permanent connection, or by means of any other mechanism which can provide access.

7. A graft according to claims 1 to 6, where the cuff, port or ports, and expandable or deflatable inner structure are adorned with markers which may be chosen so that they are visible by various diagnostic methods.

8. A graft according to claims 1 to 7, wherein access to the port can be made temporarily or by permanent attachment to a subcutaneous connecting device.

9. A graft according to claim 8, which may be implanted into a blood vessel or any other body passageway or any other artificial body passageway or orifice by means of endoscopy, interventional radiology, or any other surgical manipulation.

10. A graft according to claim 9, wherein the expandable or deflatable inner structure is accessible through a port, which itself is accessible by means of a access device, injection needle or permanent connection.
